# EUROPÄISCHE PATENTSCHRIFT

(11) **EP 0 828 463 B1**
(45) Veröffentlichungstag und Bekanntmachung des Hinweises auf die Patenterteilung: **05.12.2001**
(21) Anmeldenummer: 96916090.2
(22) Anmeldetag: 15.05.1996
(51) Int. Cl.: A61F 2/44

(54) **HÖHENVERSTELLBARER WIRBELKÖRPERERSATZ**
HEIGHT-ADJUSTABLE ARTIFICIAL VERTEBRAL BODY
CORPS VERTEBRAL ARTIFICIEL REGLABLE EN HAUTEUR

(30) Priorität: 24.05.1995 DE 19519101
(43) Veröffentlichungstag der Anmeldung: 18.03.1998
(73) Patentinhaber: Biedermann, Lutz, 78048 Villingen-Schwenningen (DE); Harms, Jürgen, 76337 Waldbronn (DE)
(72) Erfinder: Biedermann, Lutz, 78048 Villingen-Schwenningen (DE); Harms, Jürgen, 76337 Waldbronn (DE)
(74) Vertreter: Prüfer, Lutz H., Dipl.-Phys.
(86) Internationale Anmeldenummer: EP9602092
(87) Internationale Veröffentlichungsnummer: WO9637170

(56) Entgegenhaltungen:
- EP-A- 0 693 274
- AT-B- 395 524
- DE-A- 4 409 392
- DE-C- 4 323 034
- US-A- 4 657 550

## Beschreibung

Die Erfindung betrifft einen höhenverstellbaren wirbelkörperersatz.

Aus der DE-GM-91 07 494.0 ist ein höhenverstellbarer Wirbelkörperersatz mit einem Gewindestab, der in seiner Mitte einen Abschnitt zum Ineingriffbringen mit einem Schraubenschlüssel zum Verdrehen des Gewindestabes, an dem daran anschließenden einen Abschnitt ein Linksgewinde und an dem davon gegenüberliegenden zweiten Abschnitt ein Rechtsgewinde aufweist. Mit jedem Gewindeabschnitt ist jeweils ein ein entsprechendes Gewinde aufweisendes Stützelement verbunden. Das Stützelement weist auf der freien Stirnfläche in achsenparalleler Richtung hervorstehende Dornen auf. Andere höhenverstellbare Wirbelkörperimplantate sind in der US-A-4,657,550 und der DE-C-30 23 942 beschrieben.

Aus der AT-B 395 524 ist ein höhenverstellbarer Wirbelkörperersatz bekannt. Dieser weist eine Gewindehülse auf, die an einer Seite ein Rechtsgewinde und an der anderen ein Linksgewinde besitzt. Diese Gewindehülse sind von beiden Seiten her Gewindebolzen eingedreht, die an ihren freien äußeren Stirnseiten zackenartige Vorsprünge, insbesondere in Form von kleinen Pyramiden aufweisen.

Aufgabe der Erfindung ist es, einen höhenverstellbaren Wirbelkörperersatz zu schaffen, der mechanisch einfach ausgebildet und einfach bedienbar ist und der ein gutes Verwachsen ermöglicht.

In der älteren, nicht vorveröffentlichten Europäischen Patentanmeldung entsprechend der EP-A-0 693 174, die für die Vertragsstaaten BE, DE, FR, GB, IT, NL benannt ist, ist ein höhenverstellbarer Wirbelkörperersatz mit einem hülsenartigen Mittelteil, welches angrenzend an seinen ersten Rand ein Linksgewinde und angrenzend an seinen zweiten Rand ein Rechtsgewinde aufweist, beschrieben. Der Wirbelkörperersatz weist ein hohlzylindrisches erstes Teil mit einer Wandung mit einer Mehrzahl von Ausnehmungen und ein hohlzylindrisches zweites Teil mit einer Wandung mit einer Mehrzahl von Ausnehmungen auf, wobei das erste Teil ein mit dem Linksgewinde zusammenwirkendes Gewinde und das zweite Teil ein mit dem Rechtsgewinde zusammenwirkendes Gewinde besitzt. Mindestens eines der Teile weist an seinem freien Ende eine Mehrzahl von Schneiden oder Spitzen auf.

Diese Aufgabe wird durch den in Anspruch 1 beschriebenen höhenverstellbaren Wirbelkörperersatz gelöst. Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Seitenansicht in Explosionsdarstellung einer ersten Ausführungsform;
- Fig. 2: ein bearbeitetes Detail aus Fig. 1;
- Fig. 3: eine teilweise geschnitten dargestellte abgewandelte Ausführungsform; und
- Fig. 4: eine Seitenansicht einer Ausführungsform in zwei verschiedenen Einstellungen.

Der höhenverstellbare Wirbelkörperersatz weist ein zylindrisch ausgebildetes Mittelteil 1 auf. Dieses weist in axialer Richtung gesehen in der Mitte einen erhabenen Ring 2 und an seinen daran angrenzenden Abschnitten auf beiden Seiten Gewindeabschnitte 3 und 4 auf. Dabei ist der eine Abschnitt 3 als Linksgewinde und der andere Abschnitt 4 als Rechtsgewinde ausgebildet. Der Ring 2 weist in Umfangsrichtung gegeneinander versetzt angeordnete Bohrungen 5 auf, und die Gewindeabschnitte 3 und 4 weisen koaxial ausgerichtete rautenförmige Öffnungen 6 auf.

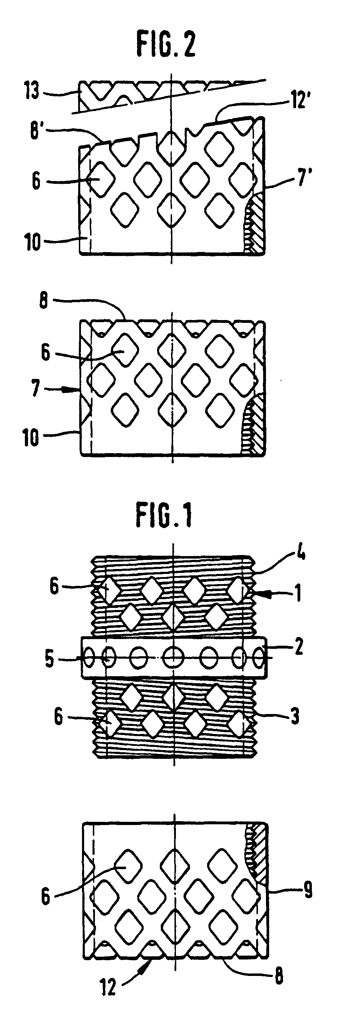

Ferner ist ein zylindrisch ausgebildetes erstes Teil 7 vorgesehen. Dieses weist auf seiner Innenseite ein mit dem Gewinde des Gewindeabschnittes 4 zusammenwirkendes Gewinde auf und ist im Betrieb auf dem Gewindeabschnitt 4 aufgeschraubt. Die Wandung ist aus einem in Umfangsrichtung und in Axialrichtung gegeneinander versetzt angeordneten Rauten 6 aufweisendem Material gebildet. An seinem dem Ring 2 abgewandten freien Ende weist das erste Teil eine Mehrzahl von in Umfangsrichtung voneinander einen Abstand aufweisende Zacken auf. Die Zacken sind, wie am besten aus Fig. 1 ersichtlich ist, dadurch gebildet, daß der Rand in Umfangsrichtung entlang der Mittellinie einer Schicht der Rauten 6 gelegt ist.

Das zweite Teil 9 unterscheidet sich gegenüber dem ersten Teil nur dadurch, daß sein Innengewinde so ausgebildet ist, daß es mit dem Gewinde des Gewindeabschnittes 3 zusammenwirkt. Das zweite Teil ist im Betrieb auf den Gewindeabschnitt 3 aufgeschraubt.

Die rautenförmigen Öffnungen 6 in den beiden Gewindeabschnitten 3 und 4 haben vorzugsweise die gleichen Größenverhältnisse und Anordnungsverhältnisse wie die Rautenanordnungen des ersten und des zweiten Teiles. Der Außendurchmesser des Ringes 2 ist im wesentlichen gleich dem Außendurchmesser des ersten bzw. zweiten Teiles 7, 9 gewählt.

Als Material für den Platzhalter wird vorzugsweise Titanblech bzw. Titanrohr gewählt, auf jeden Fall aber ein biokompatibles Material.

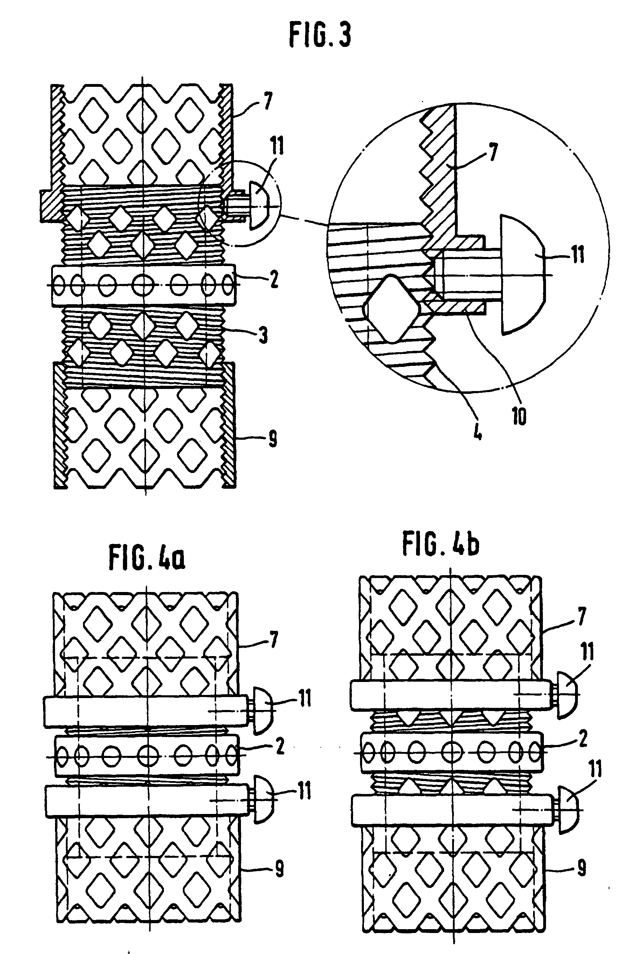

Im Betrieb wird der so beschriebene Wirbelkörperersatz in dem zusammengeschraubten Zustand einfach zwischen die abzustützenden Teile eingesetzt und durch Drehen an dem Ring 2 auf die richtige Höhe eingestellt. In Fig. 4a ist der Wirbelkörperersatz nahezu vollständig in die Ausgangsstellung auf die geringste Höhe zusammengeschraubt, während die Höhe in der in Fig. 4b gezeigten Stellung durch Auseinanderschrauben vergrößert ist. Durch die Zacken 8 erfolgt ein drehstabiler Eingriff mit den benachbarten Teilen.

Durch die Ausnehmungen 5 und 6 erfolgt ein gutes Durchwachsen mit im Inneren des hohlen Wirbelkörperersatzes anzubringender Knochensubstanz.

In Fig. 3 ist eine Ausführungsform einer Gewindetorsionssicherung für den oben beschriebenen Wirbelkörperersatz gezeigt. An dem Ring 2 zugewandten Ende 10 des ersten Teiles 7 ist eine Kopf- oder Madenschraube 11 in radialer Richtung so angeordnet, daß diese in der in Fig. 3 gezeigten Weise zur Arretierung mit dem Gewinde des zugehörigen Gewindeabschnittes des Mittelteiles in Eingriff gelangt und eine Fixierung bewirkt. Die Madenschraube ist, wie aus Fig. 3 ersichtlich ist, in einem in dem Mantel des ersten Teiles vorgesehenen Gewinde geführt.

Wie am besten aus Fig. 2 ersichtlich ist, ermöglicht die Ausbildung des jeweiligen Mantels der Teile 7, 7', 9 es, eine einfache Anpassung der Neigung der mit den benachbarten Wirbelkörpern in Eingriff zu bringenden Endflächen 12, indem mit einer geeigneten Blechschere entsprechend der gewünschten Neigung ein Teil 13 abgetrennt und somit eine geneigte Kontaktfläche 12' geschaffen wird. Durch die Auswahl des Materials weist auch die neue Kontaktfläche 12' entsprechende Zacken 8' auf.

Im Betrieb erfolgt nach der Höheneinstellung mittels Verdrehens des Mittelteiles 1 über den Ring 2 ein Arretieren der Schrauben 11, so daß eine stabile und sich nicht selbst lösende Fixierung erreicht ist.
¹ Translator's Note: this is a literal translation of the German text, which must be incomplete. Possible meanings, if a word were inserted, could be "at their first edge remote from the free end" or "at their first edge opposite the free end" or similar.
² Translator's Note: the same problem occurs here as in Claim 1 for the other contracting states.

## Patentansprüche (Patentansprüche für folgende(n) Vertragsstaat(en): AT, CH, LI, ES)

1. Höhenverstellbarer Wirbelkörperersatz mit einem hülsenartigen Mittelteil (1), welches angrenzend an seinen ersten Rand ein Linksgewinde und angrenzend an seinen zweiten Rand ein Rechtsgewinde aufweist,
mit einem zylindrischen ersten Teil (7) mit einer Wandung und einem zylindrischen zweiten Teil (9) mit einer Wandung, wobei das erste Teil (7) ein mit dem Linksgewinde zusammenwirkendes Gewinde und das zweite Teil (9) ein mit dem Rechtsgewinde zusammenwirkendes Gewinde und
beide Teile an ihrem freien Ende ersten Rand jeweils eine Mehrzahl von Zacken (8) aufweisen, und wobei die Wandungen des ersten Teiles (7) und des zweiten Teiles (9) mit einer Mehrzahl von Ausnehmungen (6) versehen sind.

2. Höhenverstellbarer Wirbelkörperersatz nach Anspruch 1, **dadurch gekennzeichnet, daß** das Gewinde jedes der Teile (7, 9) an dem dem ersten Rand gegenüberliegenden zweiten Rand vorgesehen ist.

3. Höhenverstellbarer Wirbelkörperersatz nach Anspruch 1 oder 2, **dadurch gekennzeichnet, daß** die Gewinde des Mittelteiles (1) als Außengewinde und die der Teile als Innengewinde ausgebildet sind.

4. Höhenverstellbarer Wirbelkörperersatz nach einem der Ansprüche 1 bis 3, **dadurch gekennzeichnet, daß** das Mittelteil (1) zwischen den Gewindeabschnitten (3, 4) einen Abschnitt (2) zum Drehen des Mittelteiles (1) relativ zu den beiden Teilen (7, 9) aufweist.

5. Höhenverstellbarer Wirbelkörperersatz nach einem der Ansprüche 1 bis 4, **dadurch gekennzeichnet, daß** eine Einrichtung (11, 12) zum Arretieren des Mittelteiles (1) gegen Relativdrehung zu wenigstens einem Teil (7, 8) vorgesehen ist.

6. Höhenverstellbarer Wirbelkörperersatz nach einem der Ansprüche 1 bis 5, **dadurch gekennzeichnet, daß** die Ausnehmungen (5, 6) in der Wandung der Teile (7, 9) bzw. des Mittelteiles als sich im wesentlichen in Axialrichtung der Hohlkörper erstreckende Vierecke bzw. Rauten (8) ausgebildet sind.

7. Höhenverstellbarer Wirbelkörperersatz nach einem der Ansprüche 1 bis 6, **dadurch gekennzeichnet, daß** als Material Titanblech bzw. Titanrohr gewählt ist.

## Patentansprüche (Patentansprüche für folgende(n) Vertragsstaat(en): DE, FR, GB)

1. Höhenverstellbarer Wirbelkörperersatz mit einem hülsenartigen Mittelteil (1), welches angrenzend an seinen ersten Rand ein Linksgewinde und angrenzend an seinen zweiten Rand ein Rechtsgewinde aufweist,
mit einem zylindrischen ersten Teil (7) mit einer Wandung mit einer Mehrzahl von Ausnehmungen (6) und einem zylindrischen zweiten Teil (9) mit einer Wandung mit einer Mehrzahl von Ausnehmungen (6), wobei das erste Teil (7) ein mit dem Linksgewinde zusammenwirkendes Gewinde und das zweite Teil (9) ein mit dem Rechtsgewinde zusammenwirkendes Gewinde und
beide Teile an ihrem freien Ende ersten Rand jeweils eine Mehrzahl von Zacken (8), aufweisen und daß der Wirbelkörperersatz aus Titan bzw. Titanrohr gebildet ist.

2. Höhenverstellbarer Wirbelkörperersatz nach Anspruch 1, **dadurch gekennzeichnet, daß** das Gewinde jedes der Teile (7, 9) an dem dem ersten Rand gegenüberliegenden zweiten Rand vorgesehen ist.

3. Höhenverstellbarer Wirbelkörperersatz nach Anspruch 1 oder 2, **dadurch gekennzeichnet, daß** die Gewinde des Mittelteiles (1) als Außengewinde und die der Teile als Innengewinde ausgebildet sind.

4. Höhenverstellbarer Wirbelkörperersatz nach einem der Ansprüche 1 bis 3, **dadurch gekennzeichnet, daß** das Mittelteil (1) zwischen den Gewindeabschnitten (3, 4) einen Abschnitt (2) zum Drehen des Mittelteiles (1) relativ zu den beiden Teilen (7, 9) aufweist.

5. Höhenverstellbarer Wirbelkörperersatz nach einem der Ansprüche 1 bis 4, **dadurch gekennzeichnet, daß** eine Einrichtung (11, 12) zum Arretieren des Mittelteiles (1) gegen Relativdrehung zu wenigstens einem Teil (7, 8) vorgesehen ist.

6. Höhenverstellbarer Wirbelkörperersatz nach einem der Ansprüche 1 bis 5, **dadurch gekennzeichnet, daß** die Ausnehmungen (5, 6) in der Wandung der Teile (7, 9) bzw. des Mittelteiles als sich im wesentlichen in Axialrichtung der Hohlkörper erstreckende Vierecke bzw. Rauten (8) ausgebildet sind.

## Claims (Claims for the following Contracting State(s): AT, CH, LI, ES)

1. A height-adjustable artificial vertebral body having a sleeve-like central part (1) which has adjacent to its first edge a left-hand thread and adjacent to its second edge a right-hand thread,
having a cylindrical first part (7) with a wall and a cylindrical second part (9) with a wall, the first part (7) being a thread co-operating with the left-hand thread and the second part (9) being a thread co-operating with the right-hand thread, and both parts having at their free end first edge¹ in each case a plurality of jagged formations (8),
**characterised in that** the walls of the first part (7) and the second part (9) are provided with a plurality of cutouts (6).

2. A height-adjustable artificial vertebral body according to Claim 1, **characterised in that** the thread of each of the parts (7, 9) is provided at the second edge opposite the first edge.

3. A height-adjustable artificial vertebral body according to Claim 1 or 2, **characterised in that** the threads of the central part (1) are constructed as external threads and those of the parts are constructed as internal threads.

4. A height-adjustable artificial vertebral body according to one of Claims 1 to 3, **characterised in that** the central part (1) has between the threaded sections (3, 4) a section (2) for turning the central part (1) relative to the two parts (7, 9).

5. A height-adjustable artificial vertebral body according to one of Claims 1 to 4, **characterised in that** a device (11, 12) for locking the central part (1) to prevent relative rotation with respect to at least one part (7, 8) is provided.

6. A height-adjustable artificial vertebral body according to one of Claims 1 to 5, **characterised in that** the cutouts (5, 6) in the wall of the parts (7, 9) and of the central part are constructed as squares or lozenges (8) extending substantially in the axial direction of the hollow bodies.

7. A height-adjustable artificial vertebral body according to one of Claims 1 to 6, **characterised in that** sheet titanium or tubular titanium is selected as the material.

## Claims (Claims for the following Contracting State(s): DE, FR, GB)

1. A height-adjustable artificial vertebral body having a sleeve-like central part (1) which has adjacent to its first edge a left-hand thread and adjacent to its second edge a right-hand thread,
having a cylindrical first part (7) with a wall having a plurality of cutouts (6) and a cylindrical second part (9) with a wall having a plurality of cutouts (6), the first part (7) being a thread co-operating with the left-hand thread and the second part (9) being a thread co-operating with the right-hand thread, and
both parts having at their free end first edge² in each case a plurality of jagged formations (8), and in that the artificial vertebral body is formed from titanium or tubular titanium.

2. A height-adjustable artificial vertebral body according to Claim 1, **characterised in that** the thread of each of the parts (7, 9) is provided at the second edge opposite the first edge.

3. A height-adjustable artificial vertebral body according to Claim 1 or 2, **characterised in that** the threads of the central part (1) are constructed as external threads and those of the parts are constructed as internal threads.

4. A height-adjustable artificial vertebral body according to one of Claims 1 to 3, **characterised in that** the central part (1) has between the threaded sections (3, 4) a section (2) for turning the central part (1) relative to the two parts (7, 9).

5. A height-adjustable artificial vertebral body according to one of Claims 1 to 4, **characterised in that** a device (11, 12) for locking the central part (1) to prevent relative rotation with respect to at least one part (7, 8) is provided.

6. A height-adjustable artificial vertebral body according to one of Claims 1 to 5, **characterised in that** the cutouts (5, 6) in the wall of the parts (7, 9) and of the central part are constructed as squares or lozenges (8) extending substantially in the axial direction of the hollow bodies.

## Revendications (Revendications pour l'(les) Etat(s) contractant(s) suivant(s): AT, CH, LI, ES)

1. Corps vertébral artificiel réglable en hauteur formé par une pièce centrale (1) en forme de manchon, qui, dans une zone adjacente à son premier bord, comporte un filet à gauche et, dans une zone adjacente à son deuxième bord, comporte un filet à droite, par une première pièce (7) cylindrique munie d'une paroi et par une deuxième pièce (9) cylindrique munie d'une paroi, sachant que la première pièce (7) comporte un filet concourant avec le filet à gauche et la deuxième pièce (9) comporte un filet concourant avec le filet à droite et les deux pièces sont munies chacune sur l'extrémité libre de leur premier bord d'une pluralité de pointes (8), **caractérisé en ce que** les parois de la première pièce (7) et de la deuxième pièce (9) sont munies d'une pluralité d'évidements (6).

2. Corps vertébral artificiel réglable en hauteur selon la revendication 1, **caractérisé en ce que** le filet de chacune des pièces (7, 9) est prévu sur le deuxième bord en regard du premier bord.

3. Corps vertébral artificiel réglable en hauteur selon la revendication 1 ou 2, **caractérisé en ce que** les filets de la pièce centrale (1) sont conçus sous forme de filet extérieur et ceux des pièces sont conçus sous forme de filets intérieurs.

4. Corps vertébral artificiel réglable en hauteur selon l'une quelconque des revendications 1 à 3, **caractérisé en ce que** la pièce centrale (1) comporte, entre les parties filetées (3, 4), une partie (2) pour faire tourner la pièce centrale (1) par rapport aux deux autres pièces (7, 9).

5. Corps vertébral artificiel réglable en hauteur selon l'une quelconque des revendications 1 à 4, **caractérisé en ce qu'**il est prévu un dispositif (11, 12) destiné à bloquer la pièce centrale (1) pour empêcher un mouvement de rotation par rapport à au moins une pièce (7, 8).

6. Corps vertébral artificiel réglable en hauteur selon l'une quelconque des revendications 1 à 5, **caractérisé en ce que** les évidements (5, 6) dans la paroi des pièces (7, 9) ou de la pièce centrale sont conçus sous forme de rectangles ou losanges (8) qui s'étendent sensiblement dans le sens axial des corps creux.

7. Corps vertébral artificiel réglable en hauteur selon l'une quelconque des revendications 1 à 6, **caractérisé en ce que** le matériau choisi est une plaque en titane ou un tube en titane.

## Revendications (Revendications pour l'(les) Etat(s) contractant(s) suivant(s): DE, FR, GB)

1. Corps vertébral artificiel réglable en hauteur formé par une pièce centrale (1) en forme de manchon, qui, dans une zone adjacente à son premier bord, comporte un filet à gauche et, dans une zone adjacente à son deuxième bord, comporte un filet à droite, par une première pièce (7) cylindrique, munie d'une paroi contenant une pluralité d'évidements (6); et par une deuxième pièce (9) cylindrique, munie d'une paroi contenant une pluralité d'évidements (6), dans lequel corps vertébral la première pièce (7) comporte un filet concourant avec le filet à gauche et la deuxième pièce (9) comporte un filet concourant avec le filet à droite et les deux pièces sont munies chacune sur l'extrémité libre de leur premier bord d'une pluralité de pointes (8) et le corps vertébral artificiel est réalisé en titane ou formé dans un tube en titane.

2. Corps vertébral artificiel réglable en hauteur selon la revendication 1, **caractérisé en ce que** le filet de chacune des pièces (7, 9) est prévu sur le deuxième bord en regard du premier bord.

3. Corps vertébral artificiel réglable en hauteur selon la revendication 1 ou 2, **caractérisé en ce que** les filets de la pièce centrale (1) sont conçus sous forme de filet extérieur et ceux des pièces sont conçus sous forme de filets intérieurs.

4. Corps vertébral artificiel réglable en hauteur selon l'une quelconque des revendications 1 à 3, **caractérisé en ce que** la pièce centrale (1) comporte, entre les parties filetées (3, 4), une partie (2) pour faire tourner la pièce centrale (1) par rapport aux deux autres pièces (7, 9).

5. Corps vertébral artificiel réglable en hauteur selon l'une quelconque des revendications 1 à 4, **caractérisé en ce qu'**il est prévu un dispositif (11, 12) destiné à bloquer la pièce centrale (1) pour empêcher un mouvement de rotation par rapport à au moins une pièce (7, 8).

6. Corps vertébral artificiel réglable en hauteur selon l'une quelconque des revendications 1 à 5, **caractérisé en ce que** les évidements (5, 6) dans la paroi des pièces (7, 9) ou de la pièce centrale sont conçus sous forme de rectangles ou losanges (8) qui s'étendent sensiblement dans le sens axial des corps creux.
